# EUROPÄISCHE PATENTSCHRIFT

(11) **EP 2 100 592 B1**
(45) Veröffentlichungstag und Bekanntmachung des Hinweises auf die Patenterteilung: **10.08.2011**
(21) Anmeldenummer: 09154899.0
(22) Anmeldetag: 11.03.2009
(51) Int. Cl.: A61K 8/97, A61Q 19/08, A61K 8/99

(54) **Kosmetikum mit Anti-Alterungswirkung**
Cosmetic product with anti-aging effect
Produit cosmétique doté d'un effet anti-vieillissement

(30) Priorität: 11.03.2008 DE 102008014289
(43) Veröffentlichungstag der Anmeldung: 16.09.2009
(73) Patentinhaber: Coty Germany GmbH, 55116 Mainz (DE)
(72) Erfinder: Golz-Berner, Karin, 98000 Monaco (MC); Zastrow, Leonhard, 98000 Monaco (MC)
(74) Vertreter: Gulde Hengelhaupt Ziebig & Schneider

(56) Entgegenhaltungen:
- EP-A- 1 038 519
- WO-A-02/092042
- WO-A-2005/034891
- JP-A- 2006 328 024
- ANONYMOUS: "Anti-Aging Products from Pentapharm"[Online] August 2006 (2006-08), XP002532576 Gefunden im Internet: URL:http://web.archive.org/web/20060827195 410/http://www.pentapharm.com/sw190.asp> [gefunden am 2009-06-17]
- P.K.INAMDAR, R.D. YEOLE, A.B.GHOGARE, N.J. DE SOUZA: "Determination of biologically active constituents in Centella asiatica" JOURNAL OF CHROMATOGRAPHY A, [Online] Bd. 742, 1996, Seiten 127-130, XP002532570 Gefunden im Internet: URL:http://www.sciencedirect.com/science?_ ob=MImg&_imagekey=B6TG8-3V9DKMT-1G-2&_cdi= 5248&_user=987766&_orig=browse&_coverDate= 08%2F23%2F1996&_sk=992579998&view=c&wchp=d GLbVlb-zSkzk&md5=168dab03bed6dfbf1ca815027 db1779f&ie=/sdarticle.pdf> [gefunden am 2009-06-17]
- W. CISOWSKI, U. SAWICKA, M. MARDAROWICZ, M.ASZTEMBORSKA, M. LUCZKIEWICZ: "Essential Oil from Herb and Rhizome of Peucedanum ostruthium (L.Koch.) ex DC." ZEITSCHRIFT FÜR NATURFORSCHUNG, [Online] Bd. 56c, 2001, Seiten 930-932, XP002532571 Gefunden im Internet: URL:http://www.znaturforsch.com/ac/v56c/56 c0930.pdf> [gefunden am 2009-06-17]

## Beschreibung

Die Erfindung betrifft eine kosmetische Zusammensetzung, die eine verbesserte Anti-Alterungswirkung aufweist.

Der Einsatz von Pflanzenextrakten in kosmetischen Mitteln ist bekannt. Überraschend ist oft bei Kombinationen mehrerer Pflanzenextrakte die daraus resultierende Gesamtwirkung, die nicht immer den Einzelwirkungen entspricht und auch von der Art der ausgewählten Pflanzenteile und der Extraktionsmittel abhängig ist. Weiterhin ist meist nicht vorhersehbar, wie die Gesamtwirkung aussieht, wenn Extrakte mit verschiedenen Wirkungsrichtungen miteinander kombiniert werden.

Die EP 1 038 519 A1 offenbart beispielsweise die Verwendung von Siliconpfropfpolymeren als Straffungsmittel in Schlankheitsmitteln, wobei die Siliconpfropfpolymere mit straffenden Pflanzenextrakten, wie z.B. Extrakten aus Centella asiatica, Siegesbeckia oder Saccharomyces cerevisia kombiniert sein können.

Die Verwendung von Centella asiatica in Anti-Alterungs-Zusammensetzungen ist ebenfalls bereits bekannt. Die JP 2006 328024 A offenbart beispielsweise eine kosmetische Zusammensetzung zur Verminderung der Hautalterung, die hydrolysiertes Seidenprotein, Ubichinon und einen oder mehrere Pflanzenextrakte, wie z.B. Centella asiatica oder Hefe, enthält. Nach einer zweimonatigen Behandlung können Verbesserungen im Hautbild beobachtet werden, sofern die Zusammensetzungen hydrolysiertes Seidenprotein und Ubichinon enthalten.

Weiterhin beschreibt die WO2005/034891 ein Gemisch von Rosmarinextrakt und wenigstens einem weiteren Pflanzenextrakt, zu dem auch ein Centella-Extrakt gehören kann, und einem DNA-Reparaturenzym. Bei derartigen Gemischen treten Anti-Alterungswirkungen allerdings erst bei wenigstens 14-tägiger Anwendung auf. Auch das in WO02/092042 beschriebene Anti-Alterungsprodukt auf Basis von Phospholipidkomplexen von Vitis vinifera-Extrakten und Phospholipidkomplexen standardisierter Centella asiatica-Extrakte führt erwartungsgemäß zu üblichen Anti-Alterungseffekten ohne Angabe bestimmter Zeiträume, die aber normalerweise im Bereich von 30 - 40 Tagen liegen.

Der Erfindung liegt die Aufgabe zugrunde, ein Kosmetikum mit besonders schneller Anti-Alterungswirkung zu entwickeln.

Erfindungsgemäß umfasst das Kosmetikum mit Anti-Alterungswirkung ein Extraktgemisch, bestehend aus einem Extrakt von Bierhefe, einem Extrakt von Peucedanum ostruthium, das auch als Kaiserwurz bekannt ist, und einem Extrakt von Centella asiatica, deren Gehalt jeweils im Bereich von 0,1 bis 6 Gew.-% liegt, und weitere kosmetische Hilfsstoffe, Trägerstoffe und Gemische davon, wobei die Prozentangaben auf das Gesamtgewicht des Kosmetikums bezogen sind.

Überraschenderweise zeigt eine Kombination eines Sebum-regulierenden Bierhefe-Extraktes mit einem Kaiserwurzextrakt, der für seine wundheilenden und entzündungswidrigen Eigenschaften bekannt ist, zusammen mit einem Extrakt von Centella asiatica eine besonders schnelle Anti-Alterungswirkung, die bereits nach wenigen Tagen erkennbar ist. Bevorzugt tritt diese Wirkung schon nach 7 Tagen Anwendung einer entsprechenden kosmetischen Zusammensetzung auf. Diese außerordentlich schnelle Wirksamkeit war keinesfalls aus den Informationen über die Einzelbestandteile herleitbar. Anwendertests haben gezeigt, dass die erfindungsgemäße Wirkung nur mit der Kombination der drei Extrakte aus Centella asiatica, Bierhefe und Kaiserwurz zu erreichen war. Mit einem Kosmetikum, das nur Centella asiatica Extrakt oder eine Mischung aus Centella asiatica Extrakt und Bierhefe-Extrakt bzw. eine Mischung aus Centella asiatica Extrakt und Kaiserwurzextrakt enthielt, konnte keine schnelle Anti-Alterungswirkung erreicht werden. Zudem waren die insgesamt erzielten Anti-Alterungseffekte deutlich geringer als die mit der erfindungsgemäßen Extraktmischung erzielten Effekte.

Über diese signifikante Anti-Alterungswirkung hinaus lassen sich die erfindungsgemäßen Produkte besonders leicht auftragen und führen zu einer sehr glatten und angenehm weichen Haut, ohne dabei auf Erweichungsmittel zurückgreifen zu müssen. Dies ist besonders für empfindliche Haut von Vorteil.

In einer bevorzugten Anwendungsform liegt das erfindungsgemäße Anti-Alterungsmittel in einer ölfreien Körperlotion vor, die ein besonders gutes Feuchthaltevermögen zeigt. Als ölfreie Körperlotionen werden erfindungsgemäß kosmetische Zusammensetzungen bezeichnet, die keine Ölphase aufweisen, wobei optional Öle oder lipophile Substanze enthalten sein können, die aber vollständig mit der wässrigen Phase mischbar sind.

Des Weiteren bevorzugt ist, dass der Gehalt der Extrakte jeweils im Bereich von 0,5 - 5 Gew.-% liegt, insbesondere 1,2 - 5 Gew.-%.

Der Hefeextrakt ist vorzugsweise ein wässrig-alkoholischer Bierhefe-Extrakt mit der INCl-Bezeichnung Water (and) Alcohol (and) Faex (Yeast) Extract (and) Thiamin HCl (and) Pyridoxin HCl. Er besteht aus einem biologisch-organischen Hefeextrakt sowie naturidentischem Thiamin und Pyridoxin. Diese Stoffe sind für den Zellstoffwechsel von großer Bedeutung und sind an vielen biochemischen Reaktionen beteiligt, auf denen die von diesem Extrakt bekannte sebumreduzierende Wirkung beruht. Ein geeigneter Hefeextrakt ist beispielsweise der FAEX (Yeast) Extract, (Handelsname: Yeast Complex B), beispielsweise erhältlich bei CLR - Chemisches Laboratorium Dr. Kurt Richter GmbH, Deutschland.

Der Peucedanum ostruthium-Extrakt ist vorzugsweise ein wässriger Blatt-Extrakt. Ein erfindungsgemäß geeigneter Blatt-Extrakt hat z.B. die INCI-Bezeichnung Water (and) Glycerine (and) Peucedanum Ostruthium Leaf Extract, wobei die wirksamen Bestandteile 10-25 Gewichts-% des Gesamtgewichtes des Extraktes einnehmen. Er ist eine leicht viskose Flüssigkeit und aus der traditionellen Alpen-Medizin für seine wundheilenden und entzündungswidrigen Wirkungen bekannt. Der Extrakt wird auch innerlich für Verdauungsprobleme eingesetzt sowie wegen seiner stimulierenden und tonisierenden Eigenschaften. Ein erfindungsgemäß geeigneter Peucedanum ostruthium-Extrakt ist beispielsweise erhältlich bei Pentapharm, Schweiz unter dem Handelsnamen Imperatoria AO. Dabei handelt es sich um eine Mischung aus Peucedanum Ostruthium Leaf Extract (1 - 5%), Wasser (> 50%), Glycerin (25 - 50 %) und Potassium sorbate und Sodium benzoate als Konservierungsstoffe (jeweils 0,1 -1%).

Der Extrakt von Centella asiatica ist vorzugsweise ein Blattextrakt, der bei Umgebungstemperatur mittels mehrwertiger Alkohole gegebenenfalls zusammen mit Wasser gewonnen wird, z.B. mittels Glycerin oder Butylenglycol. Der Extrakt liegt als Flüssigkeit vor mit einem Wirkstoffgehalt von ca. 5 - 10 Gew.-%, bezogen auf das Gesamtgewicht des Extrakts. Ein bevorzugter Centella asiatica-Extrakt ist ein Extrakt der Blätter mit mehrwertigen Alkoholen oder einem Gemisch mehrwertiger Alkohole mit Wasser. Geeignete Extrakte sind beispielsweise extrait hydroglycolic (BG) de centella (INCI-Name: Water, Butylenglycol, Centella asiatica extract), biogreen de centella asiatica (SB) (INCI-Name: Water, Glycerine, Centella asiatica extract, citric acid) oder cosmelene de centella asiatica (INCI-Name: Butylenglycol, Centella asiatica extract) alle beispielsweise erhältlich von Greentech, Frankreich.

Erfindungsgemäß beträgt der Anteil des Extraktgemisches wenigstens 5 Gew.-%, vorzugsweise wenigstens 8 Gew.-%, bezogen auf das Gesamtgewicht des Kosmetikums. Die Extrakte können in gleichen oder in unterschiedlichen Anteilen gemischt werden. In einer bevorzugten Ausgestaltung der Erfindung liegt das Verhältnis der Extrakte von Centella asiatica zu Peucedanum zu Hefe im Bereich von 1 : 0,8 bis 2 : 0,4 bis 3,5, vorzugsweise im Bereich von 1 : 1 bis 1,5 : 0,5 bis 3, noch bevorzugter beträgt das Verhältnis der Extrakte 1 : 1 : 1.

Das erfindungsgemäße Präparat enthält weiterhin kosmetische Hilfs- und Trägerstoffe, wie sie üblicherweise in solchen Zubereitungen verwendet werden, z.B. Wasser, Konservierungsmittel, Farbstoffe, Pigmente mit färbender Wirkung, Verdickungsmittel, Duftstoffe, Alkohole, Polyole, Ester, Elektrolyte, Gelbildner, polare und unpolare Öle, Polymere, Copolymere, Emulgatoren, Tenside, Chelatoren, Wachse, Stabilisatoren, Puffer und/oder Konservierungsmittel.

Es können jedoch auch solche Hilfsstoffe zugesetzt werden, die eine zusätzliche Wirksamkeit aufweisen. Dazu gehören z.B. anorganische und organische Lichtschutzmittel, Selbstbräunungsmittel, Feuchthaltemittel, Skin conditioner, Enzyme, weitere pflanzliche Wirkstoffe, Polymere, Melanin, Antioxidationsmittel, entzündungswidrige natürliche Wirkstoffe und/oder mit Sauerstoff beladene asymmetrische lamellare Aggregate gemäß WO 94/00109 (Anspruch 1). Weitere Stoffe dieser Gruppe sind solche, die den Zustand der Haut positiv beeinflussen, insbesondere Wirkstoffe zur positiven Beeinflussung der Altershaut, insbesondere in Kombination mit Biochinonen, insbesondere Ubichinon Q10, Kreatin, Kreatinin, Camitin, Biotin, Isoflavone, Cardiolipin, Liponsäure, Anti Freezing Proteine, Arctiin, Hopfen-Extrakte und/oder Hopfen-Malz-Extrakte.

Fördernde Mittel zur Restrukturierung des Bindegewebes sind insbesondere Isoflavonoide.

Stoffe zur Unterstützung der Hautfunktionen bei trockener Haut sind insbesondere Vitamin C, Biotin, Camitin, Kreatin, Propionsäure, Grüntee-Extrakte, Eucalyptusöl, Harnstoff und Mineralsalze, insbesondere NaCl, Meeresmineralien und/oder Osmolyte.

Stoffe zur Linderung und/oder positiven Beeinflussung von irritativen Hautzuständen sind insbesondere Sericoside, verschiedene Extrakte des Süßholzes, Licochalcone, insbesondere Licochalcone A, Silymarin, Silyphos und/oder Dexpanthenol.

Modulatoren der Pigmentierung sind insbesondere Tyrosinsulfat, 8-Hexadecen-1,16-dicarbonsäure, Liponsäure, Liponamid, verschiedene Extrakte des Süßholzes, Kojisäure, Hydrochinon, Arbutin, Fruchtsäuren, insbesondere α-HydroxySäuren (AHAs), Uvae ursi, Ursolsäure, Ascorbinsäure, Grüntee-Extrakte, Aminoguanidin und/oder Pyridoxamin.

Stoffe, die eine verstärkte oder schnellere Bräunung der Haut herbeiführen, sind insbesondere Lipofuscine, Nukleinsäure, Oligonukleotide, Purine, Pyrimidine, Dihydroxyaceton, Erythrulose und/oder NO-freisetzende Substanzen.

Das erfindungsgemäße Kosmetikum kann vorteilhaft auch Antioxidationsmittel und Radikalfänger enthalten. Zu derartigen Substanzen gehören Vitamine wie Vitamin C und Derivate davon, beispielsweise Ascorbylacetat, -phosphat und -palmitat; Vitamin A und Derivate davon; Folsäure und deren Derivate; Vitamin E und deren Derivate, wie Tocopherylacetat; Flavone oder Flavonoide; Aminosäuren, wie Histidin, Glycin, Tyrosin, Tryptophan und Derivate davon; Carotinoide und Carotine, wie z.B α-Carotin, β-Carotin; Harnsäure und Derivate davon; α-Hydroxysäuren wie Citronensäure, Milchsäure, Apfelsäure; Stilbene und deren Derivate und deren Gemische.

Besonders bevorzugte Radikalfänger als Hilfsstoffe sind eine Wirkstoffzubereitung mit hohem Radikalschutzfaktor mit einem Gehalt an einem durch Extraktion der Rinde von Quebracho blanco und nachfolgender enzymatischer Hydrolyse gewonnenem Produkt, das wenigstens 90 Gew- % Proanthocyanidin-Oligomere und höchstens 10 Gew-% Gallussäure enthält, in Mikrokapseln, sowie einem durch Extraktion gewonnenen Seidenraupenextrakt, der das Peptid Cecropine, Aminosäuren und ein Vitamingemisch enthält, und einem nichtionischen, kationischen oder anionischen Hydro-Gel oder Gemisch von Hydro-Gelen, und einem oder mehreren Phospholipiden, und Wasser (WO99/66881 z.B. Wirkstoffkomplex gemäß Beispiel 1 oder 2).

Ein weiterer bevorzugter Radikalfänger ist eine Wirkstoffzubereitung, die ein in Lecithin verkapseltes Gemisch aus (in Gew.- %) 2 % Angelica archangelica-Wurzelextrakt (INCI: Angelica archangelica root extract, CAS number 84775-41-7), 2 % Pongamia pinnata-Samenextrakt (INCI: Pongamia pinnata seed extract), 2 % Camelia sinensis-Blattextrakt (INCI: Camellia sinensis Leaf Extract, CAS number 84650-60-2), 2 % Coffea arabica-Samenextrakt (INCI: Coffea arabica (coffee) seed extract, CAS number 84650-00-0) beinhaltet, wobei außerdem 8 % Glycerin, 8,25 % Alkohol denaturiert, Antioxidationsmittel und Hilfsstoffe enthalten sind.

In einer besonders bevorzugten Ausgestaltung der Erfindung enthält das Kosmetikum einen liposomenfreien Radikalfänger auf pflanzlicher Basis, noch bevorzugter ein liposomenfreies Gemisch von Pflanzenextrakten auf alkoholischer Basis.

Ein besonders geeigneter liposomenfreier Radikalfänger ist eine Wirkstoffzubereitung, die ein liposomenfreies Gemisch von Pflanzenextrakten auf alkoholischer Basis darstellt, bestehend aus 0,1 bis 2 Gew-% Extrakt grüner Kaffeebohnen, 0,1 bis 2 Gew-% Extrakt von Blättern von Camellia sinensis, 0,1 bis 2 Gew-% Extrakt von Pongamia pinnata und 0,1 bis 2 Gew-% Extrakt der Wurzeln von Angelica archangelica und dem Rest bis 100 Gew-% aus einem einwertigen C₂-C₅-Alkohol, wobei der Radikalschutzfaktor im Bereich von 1400-2900 x 10¹⁴ Radikale pro mg liegt (WO 2004/105706, Anspruch 1).

Besonders geeignete Öle sind beispielsweise Siliconöle, Mineralöle, Hydrogenated Polyisobuten, Polyisopren, Squalane, Tridecyltrimellitat, Trimethylpropantrüsostearat, Isodecylcitrat, Neopentylglycol-diheptanoat, PPG-15-stearylether, kosmetische Ester und Ether wie z.B. Glyceryl Stearate, sowie pflanzliche Öle, wie Calendulaöl, Jojobaöl, Avocadoöl, Macadamianußöl, Rizinusöl, Kakaobutter, Kokosnussöl, Maisöl, Baumwollsamenöl, Olivenöl, Palmkernöl, Rapssamenöl, Safloröl, Sesamsamenöl, Sojabohnenöl, Sonnenblumensamenöl, Weizenkeimöl, Traubenkernöl, Kukuinussöl, Distelöl, Shea-Butter, Kakaobutter, Lanolin und Gemische davon.

In einer bevorzugten Ausgestaltung der Erfindung enthält das Kosmetikum wasserlösliche Siliconöle, vorzugsweise Dimethicone Copolyol, Hydrolyzed Wheat Protein Hydroxypropyl Polysiloxane, Lauryl Methicone Copolyol, Polysiloxane, Trideceth-12, Dimethylsiloxane-Glycol Copolymer, PEG/PPG-14/4 Dimethicone, Behenoxy Dimethicone, Stearoxy Dimethicone, Dimethicone, Amodimethicone und Gemische davon.

Das erfindungsgemäße Kosmetikum kann auch übliche Feuchthaltemittel enthalten, wie Glycerin, Butylenglycol, Propylenglycol, Dipropylenglycol, Polyethyleneglycol und Gemische davon sowie Hyaluronsäure, Lactose, Glycin, Allantoin und/oder Sorbitol.

Zu geeigneten Gelbildnern als Hilfsmittel gehören Carbomer, Xanthangummi, Carrageenan, Akaziengummi, Guargummi, Agar-Agar, Alginate, Tylosen, Carboxymethylcellulose, Hydroxyethylcellulose, quaternisierte Cellulose, quaternisierter Guar, bestimmte Polyacrylate, Polyvinylalkohol, Polyvinylpyrrolidon und/oder Montmorillonit. Besonders bevorzugt sind Carbomer und/oder Xanthan Gum.

Die vorliegende Erfindung betrifft weiterhin die Verwendung des erfindungsgemäßen Kosmetikums zur Verhinderung und/oder Verzögerung der Alterung der Haut. In einer besonderen Ausgestaltung der Erfindung wird das Kosmetikum zur Verhinderung und/oder Reduzierung von Falten in der Haut, vorzugsweise in der Gesichtshaut verwendet.

Die Erfindung betrifft weiterhin die Verwendung in Produkten der dekorativen Kosmetik und/oder in Körperpflegeprodukten, vorzugsweise in Cremes, Lotionen, Gelen, Pudern, Balsam, Milch und/oder Masken.

Die Verwendung des erfindungsgemäßen kosmetischen Präparates kann z.B. erfolgen in Sonnencremes, Sonnengelen, After-sun-Produkten, Tagescremes, Nachtcremes, Masken, Körperlotionen, Reinigungsmilch, Make up's, Lippenstiften, Lippenbalsam, Körperpuder, Augenkosmetik, Haarmasken und/oder Haarspülungen. Die Herstellung derartiger Produkte erfolgt auf eine Weise, wie sie dem Fachmann auf diesem Gebiet bekannt ist.

In einer besonders bevorzugten Ausgestaltung enthält das erfindungsgemäße Kosmetikum FAEX (Yeast) Extract, Peucedanum ostruthium Extract, Centella asiatica Extract, Wasser, Feuchthaltemittel und wasserlösliche Silikonöle und optional Antioxidantien, Gelbildner, Alkohole, Puffer, Konservierungsmittel und Parfüme. Die sich ergebende Lotion hat erfindungsgemäß keine Ölphase und zeichnet sich durch ein besonders gutes Hautgefühl, hervorragende Anti-Alterungseigenschaften mit einer äußerst schnellen Wirkung sowie ein extrem langes, d.h. bis zu 24h, Feuchthaltevermögen aus.

In einer weiteren besonders bevorzugten Ausgestaltung der Erfindung enthält das erfindungsgemäße Kosmetikum FAEX (Yeast) Extract, Peucedanum ostruthium Extract, Centella asiatica Extract, Wasser, Feuchthaltemittel und pflanzliche und/oder synthetische Öle und optional Antioxidantien, Alkohole, Puffer, Konservierungsmittel und Parfüme. Die sich ergebende Creme ist fester als die erfindungsgemäße Lotion zeigt aber die gleichen Feuchthalte- und Anti-Alterungseigenschaften.

Die Erfindung soll nachstehend durch Beispiele näher erläutert werden. Alle Angaben erfolgen in Gewichtsprozent, sofern nichts anderes angegeben ist.

### Beispiel 1 Ölfreie Körperlotion I

| **Phase A** | |
|---|---|
| Wasser | q.s. ad 100 |
| Glycerin | 5,0 |
| Propylenglycol | 3,0 |
| Carbomer | 0,15 |
| Triethanolamin | 0,15 |
| Siliconöl (wasserlöslich) | 5,0 |
| Ethanol | 3,0 |
| Parfüm | 3,0 |
| Konservierungsmittel | 0,1 |
| RPF-Komplex* | 1,0 |
| FAEX (Yeast) Extract (Yeast Complex B®) | 3,0 |
| Peucedanum ostruthium Extract | 3,0 |
| Centella asiatica Extract | 3,0 |

| | |
|---|---|
| *RPF-Komplex = alkoholisches Gemisch aus 0,2 Gew-% Kaffeebohnensamen, 0,2 Gew-% Camellia sinensis Blätterextrakt, 0,2 Gew-% Ponagamia pinnata Extrakt, 0,2 Gew-% Angelikawurzelextrakt und 99,8 Gew-% Ethanol (WO 2004/105706) | |

### Beispiel 2 Ölfreie Körperlotion II

| **Phase A** | |
|---|---|
| Wasser | q.s. ad 100 |
| Glycerin | 5,0 |
| Propylenglycol | 3,0 |
| Carbomer | 0,15 |
| Triethanolamin | 0,15 |
| Siliconöl (wasserlöslich) | 5,0 |
| Ethanol | 3,0 |
| Parfüm | 3,0 |
| Konservierungsmittel | 0,1 |
| FAEX (Yeast) Extract (Yeast Complex B®) | 3,0 |
| Peucedanum ostruthium Extract | 3,0 |
| Centella asiatica Extract | 3,0 |

Die Herstellung der Lotionen von Beispiel 1 und 2 erfolgt durch Vermischung der Einzelbestandteile in der angegebenen Reihenfolge unter Rühren bei Umgebungstemperatur bis 30°C.

Die Lotionen zeigen ein sehr gutes Feuchthaltevermögen über einen längeren Zeitraum von 24 Stunden. Sie sind auf der Haut nicht klebrig und führen zu einer sehr glatten und angenehm weichen Haut mit einem hochwirksamen Anti-Falten-Effekt, der bereits nach wenigen Tagen eintritt (siehe Beispiel 5).

### Beispiel 3 Spezial-Creme mit Anti-Alterungs-Wirkung

| **Phase A** | |
|---|---|
| Wasser | q.s. ad 100 |
| Citric Acid | 0,08 |
| Disodium EDTA | 0,4 |
| Glycerin | 2,0 |
| Propylenglycol | 2,0 |

| **Phase B** | |
|---|---|
| Glyceryl Stearate | 3,0 |
| PEG-100 Stearate | 2,0 |
| Ceteareth-20 | 3,5 |
| Tri-C12-13 Alkyl Citrate | 7,5 |
| Siliconöl | 2,0 |

| **Phase C** | |
|---|---|
| RPF Komplex* | 1,0 |
| FAEX (Yeast) Extract (Yeast Complex B®) | 5,0 |
| Peucedanum ostruthium Extract | 2,5 |
| Centella asiatica Extract | 1,66 |
| Konservierungsmittel | 0,1 |
| Parfüm | 0,5 |

| | |
|---|---|
| * siehe Beispiel 1 | |

Als Siliconöl wurde beispielsweise Dimethiconol (and) Cyclopentasiloxane (and) Cyclotetrasiloxane verwendet. Die Bestandteile der Phase A wurden bei ca. 75°C miteinander vermischt. Bei einer ähnlichen Temperatur wurde die Phase B separat hergestellt und dann mit Phase A vermischt. Nach dem Abkühlen auf 35 - 40 °C erfolgte unter Rühren die Zugabe der Phase C.

Die erhaltene Creme führte bei Anwendung zu einem sehr weichen Hautgefühl und ließ sich besonders leicht auftragen. Sie war nicht klebrig oder ölig und zeigte eine überraschend schnelle Anti-Falten-Wirksamkeit.

### Beispiel 4 Tag- und Nachtcreme

| **Phase A** | |
|---|---|
| Wasser | q.s. ad 100 |
| Propylenglycol | 5,0 |

| **Phase B** | |
|---|---|
| PEG-9 Polydimethylsiloxyethyl Dimethicone & | |
| Dimethicone / Polyglycerin 3-CrossPolymer | 1,5 |
| Dipropylene Glycol | 2,3 |
| Tocopherol | 3,5 |
| Dimethiconol (and) Cyclopentasiloxane (and) | |
| Cyclotetrasiloxane | 3,0 |

| **Phase C** | |
|---|---|
| Shea Butter | 2,0 |
| **Phase D** | |
| Ethanol | 3,0 |
| Dimethicone | 3,0 |

| **Phase E** | |
|---|---|
| FAEX (Yeast) Extract (Yeast Complex B®) | 2,5 |
| Peucedanum ostruthium Extract | 5,0 |
| Centella asiatica Extract | 5,0 |
| Parfüm | 0,3 |
| Konservierungsmittel | 0,1 |

Die Bestandteile der Phase A wurden bei etwa 60°C miteinander verrührt. Ebenso wurden separat die Bestandteile der Phase B bei etwa 60°C verrührt. Beide Phasen wurden unter Rühren vermischt und das Gemisch auf etwa 40°C abgekühlt. Dann wurde die Phase C hinzugegeben, das Gemisch unter Rühren auf etwa 30°C abgekühlt und nacheinander die Phasen D und E hinzugegeben.

Man erhielt eine Creme mit einer sehr guten Feuchthalte- und Anti-AlterungsWirkung.

### Beispiel 5 Anwender-Test

Ein Anwendertest mit 36 Teilnehmerinnen im Alter von 37 bis 55 Jahren wurde durchgeführt. 12 Teilnehmerinnen (Gruppe A) trugen eine Creme I gemäß Beispiel 1 im Gesicht auf, insbesondere rund um die Augenpartie. Eine zweite Gruppe (Gruppe B, 12 Teilnehmerinnen) applizierte eine Creme II. Die Creme II enthielt die gleichen Bestandteile wie in Beispiel 1 genannt, jedoch ohne den Hefe-Extrakt und den Peucedanum ostruthium-Extrakt. Die dritte Gruppe (Gruppe C, 12 Teilnehmerinnen) applizierte eine Creme III, welche die gleichen Bestandteile wie in Beispiel 1 enthielt, jedoch ohne den Peucedanum ostruthium-Extrakt. Der Anteil der entfallenen Extrakte an der Gesamtmischung in den Cremes II und III wurde durch einen höheren Anteil an Wasser ausgeglichen.

Die Cremes wurden zweimal täglich morgens und abends mit einer durchschnittlichen Menge von 2-4 mg/cm² aufgetragen. Es erfolgte eine computerisierte Auswertung über digitalisierte Aufnahmen von den Augenwinkeln der Probandinnen. Die Faltenreduzierung der feinen Augenwinkelfalten wurde in Prozent festgestellt. Die Ergebnisse sind aus Tabelle 1 zu entnehmen.

**Tabelle 1**

| **Faltenreduzierung [%]** | | | |
|---|---|---|---|
| | Gruppe A | Gruppe B | Gruppe C |
| | (Creme I) | (Creme II) | (Creme III) |
| Behandlungsbeginn | 0 | 0 | 0 |
| nach 3 Tagen | 18-21 | 6-8 | 6-7 |
| nach 5 Tagen | 28-33 | 11-14 | 10-14 |
| nach 7 Tagen | 39-44 | 19-21 | 20-23 |
| nach 10 Tagen | 44-47 | 23-26 | 25-27 |
| nach 30 Tagen | 43-46 | 33-37 | 35-38 |

Tabelle 1 zeigt deutlich die Überlegenheit der Creme I mit der erfindungsgemäßen Kombination von drei Komponenten gegenüber der Einzelanwendung von Centella asiatica in der Creme II und der Wirkstoffkombination aus Centella asiatica und Hefe-Extrakt in der Creme III. Die Behandlung mit der Creme II führt zu einem erwarteten Anstieg der Faltenreduzierung im Zeitraum bis zu 30 Tagen von etwa 35 %. Bei der Behandlung mit der Wirkstoffkombination aus Cetella asiatica und Hefe-Extrakt in der Creme III können nach 30 Tagen bereits bis zu 38 % Faltenreduzierung erreicht werden. Allerdings ist die Verbesserung des Effektes mit Creme II und III erst nach dem 7. Tag der Behandlung zu beobachten. In den ersten Tagen der Behandlung sind die mit Creme II und Creme III erzielten Effekte gleich groß. Dagegen zeigt die erfindungsgemäße Creme I gemäß Beispiel 1 bereits in den ersten Tagen einen überraschenden Anstieg, der nach 7 - 10 Tagen bis auf etwa 45 % führt und danach nicht weiter steigt. Dies ist ein signifikantes und überraschendes Ergebnis.

## Patentansprüche

1. Kosmetikum mit Anti-Alterungswirkung, **dadurch gekennzeichnet, dass** es ein Extraktgemisch umfasst, bestehend aus einem Extrakt von Bierhefe, einem Extrakt von Peucedanum ostruthium und einem Extrakt von Centella asiatica, deren Gehalt jeweils im Bereich von 0,1 bis 6 Gew.-% liegt, und weitere kosmetische Hilfsstoffe, Trägerstoffe und Gemische davon, wobei die Prozentangaben auf das Gesamtgewicht des Kosmetikums bezogen sind.

2. Kosmetikum nach Anspruch 1, **dadurch gekennzeichnet, dass** der Gehalt der Extrakte jeweils im Bereich von 0,5 - 5 Gew.-% liegt, insbesondere im Bereich von 1,2 - 5 Gew.-%.

3. Kosmetikum nach Anspruch 1, **dadurch gekennzeichnet, dass** das Verhältnis der Extrakte von Centella asiatica zu Peucedanum zu Hefe im Bereich von 1 : 0,8 bis 2 : 0,4 bis 3,5, vorzugsweise im Bereich von 1 : 1 bis 1,5 : 0,5 bis 3 liegt und noch bevorzugter 1 : 1 : 1 beträgt.

4. Kosmetikum nach Anspruch 1, **dadurch gekennzeichnet, dass** der Anteil des Extraktgemisches wenigstens 5 Gew.-%, vorzugsweise wenigstens 8 Gew.-%, beträgt, bezogen auf das Gesamtgewicht des Kosmetikums.

5. Kosmetikum nach Anspruch 1, **dadurch gekennzeichnet, dass** das Kosmetikum einen liposomenfreien Radikalfänger auf pflanzlicher Basis enthält, noch bevorzugter ein liposomenfreies Gemisch von Pflanzenextrakten auf alkoholischer Basis.

6. Kosmetikum nach Anspruch 1, **dadurch gekennzeichnet, dass** der Hefeextrakt ein wässrig-alkoholischer Bierhefe-Extrakt mit der INCl-Bezeichnung Water (and) Alcohol (and) Faex (Yeast) Extract (and) Thiamin HCl (and) Pyridoxin HCl ist.

7. Kosmetikum nach Anspruch 1, **dadurch gekennzeichnet, dass** der Peucedanum ostruthium-Extrakt ein wässriger Blattextrakt ist.

8. Kosmetikum nach Anspruch 1, **dadurch gekennzeichnet, dass** der Centella asiatica-Extrakt ein Extrakt der Blätter mit mehrwertigen Alkoholen oder einem Gemisch mehrwertiger Alkohole mit Wasser ist.

9. Verwendung des Kosmetikums nach einem der Ansprüche 1 bis 8 zur Verhinderung und/oder Verzögerung der Alterung der Haut

10. Verwendung nach Anspruch 9 zur Verhinderung und/oder Reduzierung von Falten in der Haut, vorzugsweise in der Gesichtshaut.

11. Verwendung nach Anspruch 9 oder 10 in Produkten der dekorativen Kosmetik und/oder in Körperpflegeprodukten.

12. Verwendung nach einem der Ansprüche 9 bis 11 in Cremes, Lotionen, Gelen, Pudern, Balsam, Milch und/oder Masken.

## Claims

1. A cosmetic product with an anti-aging effect, **characterized in that** it comprises a mixture of extracts consisting of an extract of brewer's yeast, an extract of Peucedanum ostruthium and an extract of Centella asiatica, the content of each ranging from 0.1 to 6 wt.%, and further cosmetic auxiliaries, excipients and mixtures thereof, the percentages being based on the total weight of the cosmetic product.

2. The cosmetic product as claimed in claim 1, **characterized in that** the content of each extract is in the range of from 0.5 to 5 wt.%, more particularly in the range of from 1.2 to 5 wt.%.

3. The cosmetic product as claimed in claim 1, **characterized in that** the ratio of Centella asiatica extract to Peucedanum extract to yeast extract is in the range of from 1 : 0.8-2 : 0.4-3.5, preferably in the range of from 1 : 1-1.5 : 0.5-3, and more preferably is 1 : 1 : 1.

4. The cosmetic product as claimed in claim 1, **characterized in that** the proportion of the extract mixture is at least 5 wt.%, preferably at least 8 wt.%, based on the total weight of the cosmetic product.

5. The cosmetic product as claimed in claim 1, **characterized in that** the cosmetic product includes a plant-based free-radical scavenger free of liposomes, more preferably an alcohol-based plant extract mixture free of liposomes.

6. The cosmetic product as claimed in claim 1, **characterized in that** the yeast extract is an aqueous-alcoholic brewer's yeast extract having the INCl name: Water (and) Alcohol (and) Faex (Yeast) Extract (and) Thiamine HCl (and) Pyridoxine HCl.

7. The cosmetic product as claimed in claim 1, **characterized in that** the Peucedanum ostruthium extract is an aqueous leaf extract.

8. The cosmetic product as claimed in claim 1, **characterized in that** the Centella asiatica extract is an extract of leaves with polyhydric alcohols or a mixture of polyhydric alcohols and water.

9. Use of the cosmetic product as claimed in any of claims 1 to 8 for preventing and/or retarding skin aging.

10. The use as claimed in claim 9 for preventing and/or reducing wrinkles in the skin, preferably in the facial skin.

11. The use as claimed in claim 9 or 10 in decorative beauty culture products and/or body care products.

12. The use as claimed in any of claims 9 to 11 in creams, lotions, gels, powders, balms, milks and/or masks.

## Revendications

1. Produit cosmétique à effet anti-vieillissement, **caractérisé en ce que** qu'il contient un mélange d'extraits constitué d'un extrait de levure de bière, d'un extrait de Peucedanum ostruthium et d'un extrait de Centella asiatica, dont la teneur est à chaque fois comprise dans une gamme entre 0,1 et 6 % en poids et d'autres adjuvants, vecteurs et des mélanges de celles-ci, les données en pourcentage se rapportant au poids total du produit cosmétique.

2. Produit cosmétique selon la revendication 1, **caractérisé en ce que** la teneur des extraits est comprise à chaque fois dans une gamme entre 0,5 et 5 % en poids, notamment dans une gamme entre 1,2 et 5 % en poids.

3. Produit cosmétique selon la revendication 1, **caractérisé en ce que** le rapport respectif de l'extrait de Centella asiatica à l'extrait de Peucedanum et à l'extrait de levure est dans une gamme de 1 : 0,8 à 2 : 0,4 à 3,5, de préférence dans une gamme de 1 : 1 à 1,5 : 0,5 à 3 et de manière encore plus préférée est 1 : 1 : 1.

4. Produit cosmétique selon la revendication 1, **caractérisé en ce que** la proportion du mélange d'extraits est d'au moins 5 % en poids, de préférence d'au moins 8 % en poids, rapporté au poids total du produit cosmétique.

5. Produit cosmétique selon la revendication 1, **caractérisé en ce que** le produit cosmétique contient un capteur de radicaux libres sans liposomes à base de plantes, préférentiellement un mélange d'extraits alcooliques de plantes sans liposomes.

6. Produit cosmétique selon la revendication 1, **caractérisé en ce que** l'extrait de levure est un extrait de levure de bière aqueux et alcoolique ayant la désignation INCl Water (and) Alcohol (and) Faex (Yeast) Extract (and) Thiamine HCl (and) Pyridoxine HCl.

7. Produit cosmétique selon la revendication 1, **caractérisé en ce que** l'extrait de Peucedanum ostruthium est un extrait de feuille aqueux.

8. Produit cosmétique selon la revendication 1, **caractérisé en ce que** l'extrait de Centella asiatica est un extrait de feuilles avec des alcools multivalents ou un mélange de plusieurs alcools multivalents avec de l'eau.

9. Utilisation du produit cosmétique selon l'une quelconque des revendications 1 à 8 pour prévenir et/ou retarder le vieillissement de la peau.

10. Utilisation selon la revendication 9 pour prévenir et/ou réduire les rides dans la peau, de préférence dans la peau du visage.

11. Utilisation selon la revendication 9 ou 10 dans des produits de cosmétique décorative et/ou des produits de soin corporel.

12. Utilisation selon l'une quelconque des revendications 9 à 11 dans des crèmes, des lotions, des gels, des poudres, du baume, du lait et/ou des masques.
